# EUROPEAN PATENT SPECIFICATION

(11) **EP 1 858 452 B1**
(45) Date of publication and mention of the grant of the patent: **08.04.2015**
(21) Application number: 06735727.7
(22) Date of filing: 22.02.2006
(51) Int. Cl.: A61F 2/32, A61F 2/36

(54) **LONG SLEEVES FOR USE WITH STEMS**
LANGE MANSCHETTEN ZUR VERWENDUNG MIT SCHÄFTEN
MANCHONS LONGS À UTILISER AVEC DES TIGES

(30) Priority: 22.02.2005 US 655101 P
(43) Date of publication of application: 28.11.2007
(73) Proprietor: Smith & Nephew, Inc., Memphis, TN 38116 (US)
(72) Inventor: KELMAN, David, C., Collierville, TN 38017 (US); LAMBERT, Richard, D., Germantown, TN 38139 (US); BERGIN, Alisha, Southaven, Mississippi 38671 (US)
(74) Representative: Smith & Nephew
(86) International application number: PCT/US2006/006184
(87) International publication number: WO 2006/091629

(56) References cited:
- EP-A- 0 554 987
- EP-A- 0 555 004
- EP-A- 0 567 349
- EP-A- 0 962 197
- EP-A- 1 125 561
- FR-A- 2 675 042

## Description

### FIELD OF THE INVENTION

The present invention relates to implant components, and more particularly to sleeve components for use with implant stems.

### BACKGROUND

Joint replacement surgery can be performed on hips, knees, elbows, shoulders, and other joints to replace a diseased or damaged natural joint. A common joint replacement surgery is hip arthroplasty, which may replace one or both of the ball part of the femur that articulates in the hip socket and/or the hip socket itself. If the ball part of the femur needs to be replaced, a hip implant stem (also referred to as a femoral stem) is typically used, and various hip stem designs and methods are well known in the art. Regardless of what type of stem is used, it is important to secure the hip stem securely within the femoral canal for the initial fixation, as well as to promote the long-term stability of the implant.

One feature that may be provided in conjunction with various hip stems to help stabilize the stem in plane in a canal is a sleeve. Sleeves are typically provided with a bore that extends through the sleeve, and a hip stem or other implant stem is received through the bore. The proximal part of the sleeve typically has a spout or other surface that engages the proximal part of the femur for stabilization. While this mechanism for fixation is usually adequate for primary surgeries, more complex surgeries such as revision and congenital dysplasia of the hip surgeries may require alternate and better types of stem/sleeve fixation, particularly at the middle to more distal part of the bone.

Some current femoral fixation devices and techniques include a cylindrical stem that is biologically and/or bead coated. Other systems use a tapered grit-blasted stem. These cylindrical and tapered stems have had relatively good clinical success, but not without complications. Some surgeons who use traditional, conical, tapered hip stems find it difficult to recreate the femoral neck height because the stem locks into place in the femoral canal too high or too low. Difficulties in achieving reproducible reamer depth placement and variations in bone quality are other factors that result in the stem placement uncertainty. Some revision tapered hip systems offer modular necks of various heights to compensate for the height deficiency, but in some instances, offering modular necks as the only feature to compensate for such problems may increase the complexity of the surgical procedure. Cylindrical biologically and/or bead coated revision hip stems have the advantage of a more reproducible fit during the surgery, however, the biological enhancement coating in conjunction with the cylindrical shape of the implant may cause stress-shielding to the proximal bone. Unlike a tapered stem, a cylindrical stem loads the bone entirely against shear forces within the femoral canal. The inefficient loading distribution is likely to cause bone loss proximally, making future revision surgeons even more difficult.

Accordingly, when seeking support for the prosthesis more distally, the surgeon often uses a longer revision stem. With such long stems, the most distal section of the stem must be modified to accommodate the anterior bow of the femur. Examples of modifications include bowing or angling the distal section of the stem and/or adding a chamfer onto distal tip of the stem. Without modification, the stem may perforate the cortex of the femur. Both approaches and other variations, however, introduce a severe limitation in stem neck orientation. The surgeon is often unable to adequately reproduce the desired stem prosthesis version angle. Accordingly, additional hip stems for more complex surgeries are needed. EP Patent Publication 0962197 describes a prosthesis for use with a centralizer and a sleeve. The sleeve may fully enclose the distal end of the prosthesis or alternatively act as a proximal guide for the prosthesis.

### SUMMARY

The present invention is defined in claim 1. Various embodiments of the invention relate to sleeves having lengths that allow the sleeve to engage a patient's femoral intramedullary canal in a mid to distal region rather than only proximally. In preferred embodiments, the sleeve length is between about 60 millimeters to about 150 millimeters.

This deep fixation of the sleeve in the intramedullary canal allows a stem that extends through the sleeve to achieve a greater fixation more proximally than stems that have only a distal taper or bowed distal end or other feature at the distal end of the stem. This more proximal fixation provided by the long sleeve's ability to fixate in the mid to distal part of the femur may help enhance the integrity of the stem by moving loading stresses to more proximal regions of the stem, rather than distally.

Such long sleeves can provide better fixation when there is less proximal bone on which a standard stem can "grab" and/or "rest." Other embodiments of the long sleeves according to this invention may help bridge bone defects or bypass sclerotic bone. In certain embodiments, the sleeve is adapted to extend beyond the lesser trochanter, to or near or beyond a patient's isthmus and provide diaphyseal fixation.

### BRIEF DESCRIPTION OF THE DRAWINGS

FIG. 1 shows a standard length sleeve in combination with a stem.
FIG. 2 shows a long length sleeve in combination with a stem.
FIG. 3 shows a long length sleeve according to one embodiment of this invention in place in a patient's femoral bone.
FIG. 4 shows a long length sleeve according to another embodiment of this invention in place in a patient's femoral bone.
FIG. 5 shows an alternate embodiment of a long length sleeve without a spout.
FIG. 6 shows a diagram of the anatomy of the femur.

### DETAILED DESCRIPTION OF THE DRAWINGS

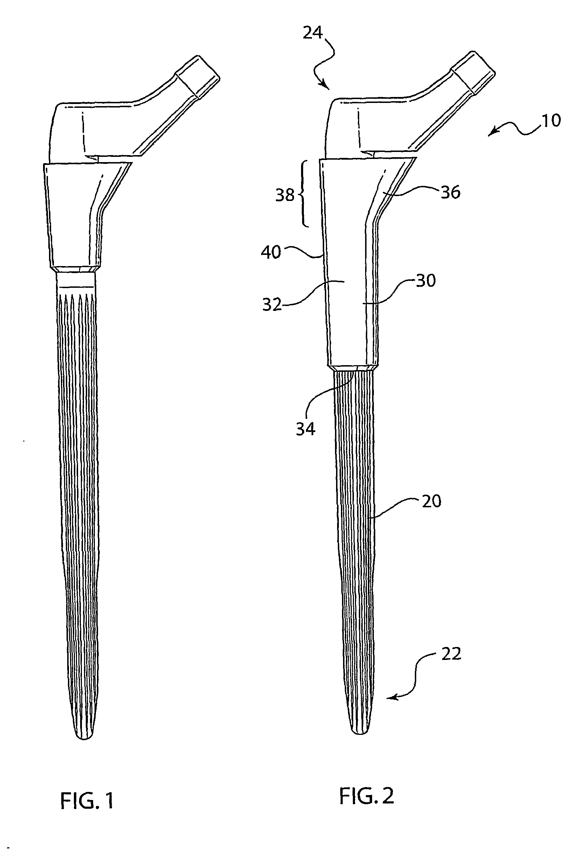

FIG. 1 shows a standard length sleeve. The standard length is typically about 30 to 40 millimeters long and fits well in a primary femoral total hip arthroplasy. In revision arthroplasty, however, a standard sleeve may not provide the support needed, for example, where there is minimal to no proximal bone. In such cases, it may be desirable to use a long sleeve, according to various embodiments of this invention.

For example, FIG. 2 shows an implant assembly 10 having a stem 20 and a sleeve 30. The sleeve 30 comprises a body 32 that has a bore 34 extending therethrough. The bore 34 provides an inner surface that can engage the outer surface of the stem 20. The sleeve 30 may slide along the stem 20 until the two components engage securely, using any appropriate method, such as frictional engagement, tapered engagement, press fit engagement, interlocking features, or any other method. The sleeve 30 may slide up from the distal portion 22 of stem or over the proximal portion 24 of the stem 20 (particularly if a modular neck is used in connection with the stem/sleeve combination assembly 10).

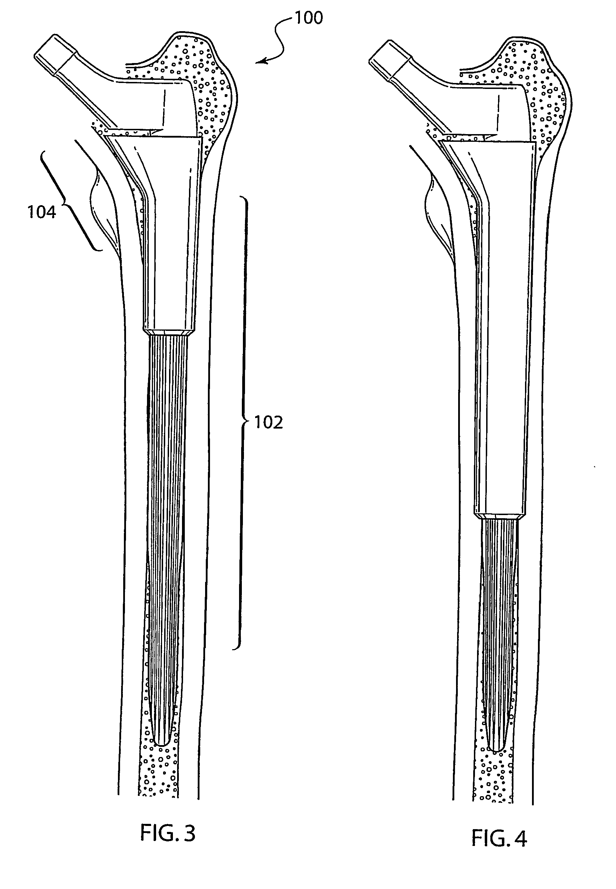

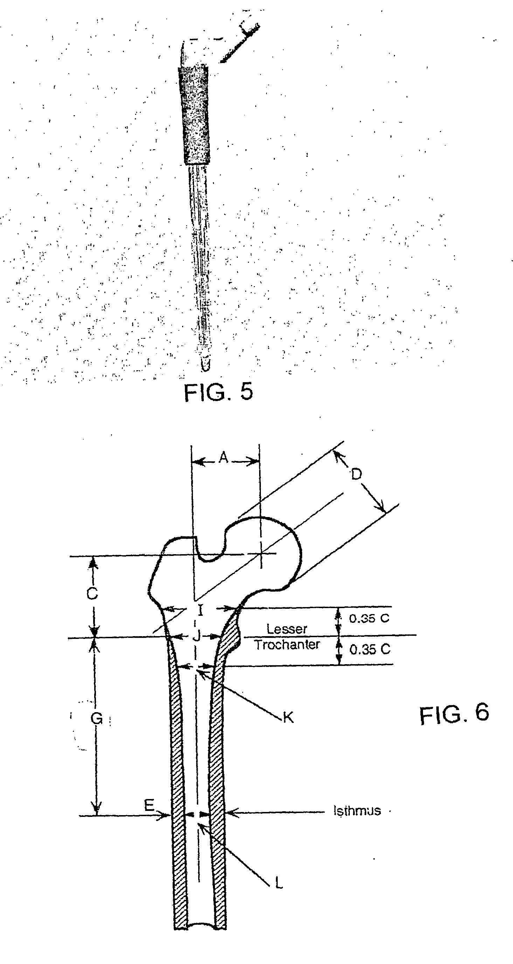

The sleeve 30 preferably has a spout 36 that protrudes from a proximal porton 38 of sleeve. Spout 36 is typically a protruding section exending from the upper portion 38 of sleeve body 32 that can be seated in the proximal portion of the femur 100, as shown in FIG. 3. The outer surface 40 of sleeve 30 can be formed in any desired geometry to compensate for bone loss. For example, it may be cylindrical, tapered, oval, oblong, elliptical, more pronouced on one side, symmetrical, non-symmetrical, any combination of these options, or any other desired shape. In a preferred embodiment, the sleeve is generally cone shaped, but it may also have cylindrical sections in parts or throughout, as shown in FIG. 2. Geometric irregularities, such as spouts or bumps, may also be offered to fill varying geometries of various bones. If provided, the spout 36 may also have different shapes, angles, extension lengths, or any combination thereof. It is also possible to provide a sleeve without a spout, as shown in FIG. 5

Outer surface 40 of sleeve 30 may optionally be provided with a bone-engaging feature that may provide the sleeve and stem combination with more stability. The bone engaging feature may include a surface that is treated with one or more of grit blasting, bead coating, fluting, porous coating, absorbable materials, surface enhancing materials or any other appropriate surface treatment. It may also be possible to provide terraced or stepped portions on spout 36.

Importantly, the sleeve 30 has a length that allows it to engage a patient's femur 100 in a mid to distal region 102 of the femoral intramedullary canal rather than only proximally 104, as shown in FIG. 3. In a particularly preferred embodiment, the sleeve length is between about 60 millimeters to about 150 millimeters. The general goal sought to be accomplished by the long sleeves of this invention is to provide a sleeve that is adapted to extend beyond the lesser trochanter, to or near or beyond a patient's isthmus area for diaphyseal fixation, which can vary from patient to patient. (Accordingly, varied sleeves of this invention may be provided as part of a kit, described in more detail below.) In some instances, a long sleeve that is greater than about 60 millimeters will provide good support for patients having minimal to no proximal bone because sleeves that reach further down the femoral canal provide improved support.

A particularly preferred sleeve according to various embodiments is adapted to engage the portion of bone between the lesser trochanter and the isthmus of the femur bone. (FIG. 6 shows an example of the anatomy of the femur and how these references points relate to one another.) In FIG. 6, the lesser trochanter is indicated as "J" and the isthmus is indicated as "L." The portion between these reference points is indicated as "G." In some embodiments, the sleeve extends between about 10% - 50% or more into the area between the lesser trochanter and the isthmus. For example, the sleeve may extend 10%, 20%, 30%, 40%, or 50% or more into the area G.

Long sleeves according to various embodiments of this invention may be manufactured and used in a manner similar to those used to manufacture and use current standard sleeves. For example, during use, the portion of the bone to receive the long sleeve is prepared using instruments such as reamers or broaches. During the preparation of the femur, a ledge is normally created by the reamer, which helps support the stem in an axial direction. The surgeon should next assess what type of long sleeve would fit best. Various long sleeves described herein may be provided as a part of kit that has a plurality of sleeves, wherein the plurality of sleeves are provided in differing, lengths or widths or both. The sleeves may also be provided with varying spout shapes and types (or sleeves without spouts), varying stem/sleeve connection mechanisms, or any other feature that will provide more options to a surgeon during a procedure. For example, a kit according to one embodiment of the invention could include one or more long sleeves provided in (a) differing lengths or widths or both; (b) varying spout shapes, angles, extension lengths, and types, or combinations thereof (as well as sleeves without spouts); (c) varying stem connection mechanisms; or (d) any combination thereof. The kit may also include a plurality of stems having varying lengths or fixation features. Further kits may include (a) one or more of the above described sleeves having varying features, (b) one or more stems having varying lengths or fixation features; and (c) one or more acetabular cup assemblies. Head components adapted to cooperate with the stem and the acetabular cup assembly may also be provided.

Once chosen, the sleeve may be press fit into the surgically prepared femur. The stem may then be inserted through the sleeve and locked into place by intimate contact between the distal bone and engagement of the sleeve (via any of the stem/sleeve connection mechanisms described above). Another implantation method may include pre-assembling the stem and sleeve combination together prior to implantation. One advantage of using a long sleeve is that it provides the ability to achieve fixation as proximal as possible, as opposed to relying on stems having angles or bows to secure the stem distally. Maintaining proximal fixation enhances the integrity of the stem by moving loading stresses to more proximal regions of the stem. As discussed, some currently available stems only feature a distal taper at the end of stem (which engages the bone at the bottom of the stem) to provide the fixation. This creates a flagpole effect, creating high stress at the mid-stem region, which ultimately causes the stem to fracture.

By contrast, significant decreases in stem stresses have been shown when proximal support is enhanced by adding support structures such as long sleeves. Long sleeves work by engaging bone more proximally, that is, more proximally than distal stem tapers or other distal stem fixation options, but deeper in the bone than typical bone sleeves that only rest on and extend into the proximal-most part of the bone. Long sleeves have been found to decrease stem stress and possibly increase the life of the implant.

Changes and modifications, additions and deletions may be made to the embodiments recited above and shown in the drawings without departing from the scope of the invention as defined by the following claims.

## Claims

1. A long sleeve (30) for use with a femoral implant stem (20), the long sleeve (30) comprising a sleeve body (32) with a bore (34) formed through the body (32) that is adapted to receive the implant stem (20), such that the stem (20) extends through and out of the sleeve (30), **characterized in that** the sleeve body (32) has a length that allows it to engage a patient's femoral intramedullary canal in a mid to distal region rather than only proximally and **in that** the femoral intramedullary canal has a lesser trochanter and an isthmus, and wherein the sleeve is adapted to extend past the lesser trochanter toward the isthmus.

2. The sleeve (30) of claim 1, wherein the sleeve body (32) length is between about 60 millimeters to about 150 millimeters.

3. The sleeve (30) of claim 1, wherein the sleeve body (32) is longer than about 60 millimeters.

4. The sleeve (30) of claim 1, wherein the sleeve (30) is adapted to extend to or near or beyond a patient's isthmus for diaphyseal fixation.

5. The sleeve (30) of claim 1, further comprising an implant stem (20) to be received by the bore.

6. The sleeve (30) of claim 1, wherein the sleeve (30) comprises a bone-engaging feature.

7. The sleeve (30) of claim 6, wherein the bone engaging feature comprises one or more of grit blasting, bead coating, fluted, porous coated, absorbable materials, surface enhancing materials, or any combination thereof.

8. The sleeve (30) of claim 1, wherein the sleeve is cylindrical, tapered, oval, oblong, or elliptical.

9. The sleeve (30) of claim 1, wherein the sleeve (30) is adapted to extend about 10% - 50% into an area between the lesser trochanter and the isthmus.

10. The sleeve (30) of claim 9, wherein the sleeve (30) extends about 20% past the lesser trochanter.

11. The sleeve (30) of claim 9, wherein the sleeve (30) extends about 30% past the lesser trochanter.

12. The sleeve (30) of claim 9, wherein the sleeve (30) extends about 40% past the lesser trochanter.

13. The sleeve (30) of claim 1, wherein the sleeve (30) extends about 50% past the lesser trochanter.

14. The sleeve (30) of claim 1, wherein the sleeve (30) locks to the stem (20) via a taper lock or a press fit lock.

15. A kit comprising a plurality of sleeves (30) according to claim 1, wherein the plurality of sleeves (30) are provided in
(a) differing lengths or widths or both;
(b) varying spout shapes, angles, extension lengths, and types, or combinations thereof;
(c) varying stem connection mechanisms; or
(d) any combination thereof.

16. The kit of claim 15, wherein the kit further comprises a plurality of stems (20) having varying lengths or fixation features.

17. A kit containing implants for use during a hip arthroplasty procedure, comprising:
(a) one or more sleeves (30) according to claim 15;
(b) one or more stems (20) having varying lengths or fixation features; and
(c) one or more acetabular cup assemblies.

18. The kit of claim 17, further comprising one or more head components adapted to cooperate with the stem and the acetabular cup assembly.

## Patentansprüche

1. Eine lange Manschette (30) zur Verwendung mit dem Schaft (20) eines femoralen Implantats, wobei die lange Manschette (30) einen Manschettenkörper (32) mit einer durch den Körper (32) gebildeten Bohrung (34) beinhaltet, die angepasst ist, um den Implantatschaft (20) aufzunehmen, so dass sich der Schaft (20) durch die Manschette (30) und aus ihr heraus erstreckt, **dadurch gekennzeichnet, dass** der Manschettenkörper (32) eine Länge aufweist, die es ihm ermöglicht, in einer mittleren bis distalen Region statt nur proximal in den femoralen intramedullären Kanal eines Patienten einzugreifen, und dadurch, dass der femorale intramedulläre Kanal einen kleinen Trochanter und einen Isthmus aufweist, und wobei die Manschette angepasst ist, um sich an dem kleinen Trochanter vorbei zum Isthmus hin zu erstrecken.

2. Manschette (30) gemäß Anspruch 1, wobei die Länge des Manschettenkörpers (32) zwischen etwa 60 Millimeter und etwa 150 Millimeter beträgt.

3. Manschette (30) gemäß Anspruch 1, wobei der Manschettenkörper (32) länger als etwa 60 Millimeter ist.

4. Manschette (30) gemäß Anspruch 1, wobei die Manschette (30) angepasst ist, um sich zur diaphysären Fixierung bis zu oder nahe dem Isthmus eines Patienten oder darüber hinaus zu erstrecken.

5. Manschette (30) gemäß Anspruch 1, die ferner einen Implantatschaft (20) zur Aufnahme durch die Bohrung beinhaltet.

6. Manschette (30) gemäß Anspruch 1, wobei die Manschette (30) ein Knocheneingriffsmerkmal beinhaltet.

7. Manschette (30) gemäß Anspruch 6, wobei das Knocheneingriffsmerkmal eines oder mehrere der Folgenden beinhaltet: Sandstrahlen, Schwallbeschichtung, gerippt, porös beschichtet, absorbierbare Materialien, Oberflächenverbesserungsmaterialien oder eine beliebige Kombination davon.

8. Manschette (30) gemäß Anspruch 1, wobei die Manschette zylindrisch, verjüngt, oval, länglich oder elliptisch ist.

9. Manschette (30) gemäß Anspruch 1, wobei die Manschette (30) angepasst ist, um sich um etwa 10 % bis 50 % in einen Bereich zwischen dem kleinen Trochanter und dem Isthmus zu erstrecken.

10. Manschette (30) gemäß Anspruch 9, wobei sich die Manschette (30) um etwa 20 % an dem kleinen Trochanter vorbei erstreckt.

11. Manschette (30) gemäß Anspruch 9, wobei sich die Manschette (30) um etwa 30 % an dem kleinen Trochanter vorbei erstreckt.

12. Manschette (30) gemäß Anspruch 9, wobei sich die Manschette (30) um etwa 40 % an dem kleinen Trochanter vorbei erstreckt.

13. Manschette (30) gemäß Anspruch 1, wobei sich die Manschette (30) um etwa 50 % an dem kleinen Trochanter vorbei erstreckt.

14. Manschette (30) gemäß Anspruch 1, wobei sich die Manschette (30) über eine Kegelsicherung oder eine Presssitzsicherung an den Schaft (20) schließt.

15. Ein Kit, das eine Vielzahl von Manschetten (30) gemäß Anspruch 1 beinhaltet, wobei die Vielzahl von Manschetten (30) wie folgt bereitgestellt sind:
(a) in unterschiedlichen Längen oder Breiten oder beiden;
(b) mit variierenden Formen, Winkeln, Erstreckungslängen und Arten der Tülle oder Kombinationen davon;
(c) mit variierenden Schaftverbindungsmechanismen; oder
(d) mit einer beliebigen Kombination davon.

16. Kit gemäß Anspruch 15, wobei das Kit ferner eine Vielzahl von Schäften (20) mit variierenden Längen oder Fixierungsmerkmalen beinhaltet.

17. Ein Kit, das Implantate zur Verwendung während eines Hüftarthroplastikverfahrens enthält, beinhaltend:
(a) eine oder mehrere Manschetten (30) gemäß Anspruch 15;
(b) einen oder mehrere Schäfte (20) mit variierenden Längen oder Fixierungsmerkmalen; und
(c) eine oder mehrere Hüftpfannenanordnungen.

18. Kit gemäß Anspruch 17, das ferner eine oder mehrere Kopfkomponenten beinhaltet, die angepasst sind, um mit dem Schaft und der Hüftpfannenanordnung zusammenzuwirken.

## Revendications

1. Un manchon long (30) destiné à être utilisé avec une tige d'implant fémoral (20), le manchon long (30) comprenant un corps de manchon (32) avec un alésage (34) formé à travers le corps (32) qui est conçu pour recevoir la tige d'implant (20), de telle sorte que la tige (20) s'étende à travers le manchon (30) et sorte de celui-ci, **caractérisé en ce que** le corps de manchon (32) a une longueur qui lui permet de se mettre en prise avec le canal intramédullaire fémoral d'un patient dans une région moyenne à distale plutôt que proximalement seulement et **en ce que** le canal intramédullaire fémoral a un petit trochanter et une isthme, et dans lequel le manchon est conçu pour s'étendre au-delà du petit trochanter vers l'isthme.

2. Le manchon (30) de la revendication 1, dans lequel la longueur de corps de manchon (32) est comprise entre environ 60 millimètres et environ 150 millimètres.

3. Le manchon (30) de la revendication 1, dans lequel le corps de manchon (32) est plus long que 60 millimètres environ.

4. Le manchon (30) de la revendication 1, le manchon (30) étant conçu pour s'étendre jusqu'à ou près de ou au-delà de l'isthme d'un patient pour une fixation diaphysaire.

5. Le manchon (30) de la revendication 1, comprenant en outre une tige d'implant (20) devant être reçue par l'alésage.

6. Le manchon (30) de la revendication 1, le manchon (30) comprenant une caractéristique de mise en prise avec un os.

7. Le manchon (30) de la revendication 6, dans lequel la caractéristique de mise en prise avec un os comprend un ou plusieurs éléments parmi un nettoyage par grenaillage, un revêtement de perle, à rainure, à revêtement poreux, des matériaux absorbables, des matériaux améliorant la surface, ou toute combinaison de ceux-ci.

8. Le manchon (30) de la revendication 1, le manchon étant cylindrique, effilé, ovale, oblong, ou elliptique.

9. Le manchon (30) de la revendication 1, le manchon (30) étant conçu pour s'étendre de 10 % à 50 % environ jusque dans une zone entre le petit trochanter et l'isthme.

10. Le manchon (30) de la revendication 9, le manchon (30) s'étendant de 20 % environ au-delà du petit trochanter.

11. Le manchon (30) de la revendication 9, le manchon (30) s'étendant de 30 % environ au-delà du petit trochanter.

12. Le manchon (30) de la revendication 9, le manchon (30) s'étendant de 40 % environ au-delà du petit trochanter.

13. Le manchon (30) de la revendication 1, le manchon (30) s'étendant de 50 % environ au-delà du petit trochanter.

14. Le manchon (30) de la revendication 1, le manchon (30) se verrouillant sur la tige (20) via un verrouillage conique ou un verrouillage par ajustage serré.

15. Un kit comprenant une pluralité de manchons (30) selon la revendication 1, dans lequel la pluralité de manchons (30) sont fournis
(a) en différentes longueurs ou largeurs, ou les deux ;
(b) en formes de bec verseur, angles, longueurs d'extension, et types variables, ou des combinaisons de ceux-ci ;
(c) en mécanismes de raccordement de tige variables ; ou
(d) en toute combinaison de ceux-ci.

16. Le kit de la revendication 15, le kit comprenant en outre une pluralité de tiges (20) ayant des longueurs ou des caractéristiques de fixation variables.

17. Un kit contenant des implants destinés à être utilisés durant une procédure d'arthroplastie de hanche, comprenant :
(a) un ou plusieurs manchons (30) selon la revendication 15 ;
(b) une ou plusieurs tiges (20) ayant des longueurs ou des caractéristiques de fixation variables ; et
(c) un ou plusieurs assemblages de cupule acétabulaire.

18. Le kit de la revendication 17, comprenant en outre un ou plusieurs composants de tête conçus pour coopérer avec la tige et l'assemblage de cupule acétabulaire.
